# EUROPÄISCHE PATENTANMELDUNG

(11) **EP 4 293 340 A1**
(43) Veröffentlichungstag der Anmeldung: **20.12.2023**
(21) Anmeldenummer: 23179691.3
(22) Anmeldetag: 16.06.2023
(51) Int. Cl.: G01N 3/08, G01N 33/34, G01N 3/14

(54) **VERFAHREN UND VORRICHTUNG ZUR MESSUNG DES DEHNUNGSVERHALTENS VON PAPIER**

(30) Priorität: 17.06.2022 AT 504312022
(71) Anmelder: Mondi AG, 1030 Wien (AT)
(72) Erfinder: Wiednig, Reinhold, 9400 Wolfsberg (AT); Taferner, Martin, 9431 Sankt Stefan (AT)
(74) Vertreter: Puchberger & Partner Patentanwälte

(57) **Zusammenfassung**

Die Erfindung betrifft ein Verfahren zur Messung des Dehnungsverhaltens von Papier und/oder eines papierähnlichen Materials, umfassend die folgenden Schritte: (a) Befestigen eines Prüfkörpers (3) zwischen zwei gegenüberliegenden Halteeinrichtungen (1, 2), wobei der Prüfkörper (3) Papier und/oder ein papierähnliches Material umfasst oder daraus besteht, (b) Einstellen einer vorbestimmten Temperatur und einer vorbestimmten relativen Luftfeuchte für die Messung, (c) Ausüben einer Zugkraft (F_{z}) auf den Prüfkörper (3), und (d) Messen der Änderung der Länge des Prüfkörpers (3) bei der auf den Prüfkörper (3) ausgeübten Zugkraft (F_{z}). Die Erfindung betrifft ferner eine Vorrichtung sowie die Verwendung einer derartigen Vorrichtung.

## Beschreibung

Die vorliegende Erfindung betrifft ein Verfahren zur Messung des Dehnungsverhaltens von Papier und/oder eines papierähnlichen Materials sowie eine Vorrichtung zur Messung des Dehnungsverhaltens von Papier und/oder eines papierähnlichen Materials. Die Erfindung betrifft gegebenenfalls auch die Verwendung einer Vorrichtung zur Messung des Dehnungsverhaltens von Papier und/oder eines papierähnlichen Materials.

Im Stand der Technik ist bekannt, die mechanischen Eigenschaften von Papier und papierähnlichen Materialien mittels unterschiedlicher Prüfverfahren zu bestimmen. Es sind verschiedene Normen bekannt, nach denen die mechanischen Eigenschaften bestimmt werden. Beispielsweise kann eine Bestimmung der Zugfestigkeit von Papier nach der Norm DIN EN ISO 1924-2 bzw. 1924-3 erfolgen. Der Einfluss der Umgebungsbedingungen wie Temperatur und Luftfeuchte auf die Materialeigenschaften ist bekannt. Daher werden Prüfungen bei einem festgelegten Normklima durchgeführt.

Es hat sich nun herausgestellt, dass durch die Messung der Papiereigenschaften bei definierten, insbesondere unterschiedlichen, Umgebungsbedingungen wertvolle Informationen erhalten werden können, die in weiterer Folge beispielsweise zur Optimierung der Herstellungs- bzw. Verarbeitungsbedingungen von Papiermaterialien verwendet werden können.

Eine Aufgabe der vorliegenden Erfindung kann also gegebenenfalls darin gesehen werden, ein Verfahren zu schaffen, das Rückschlüsse auf die Papiereigenschaften bei vorbestimmten und/oder unterschiedlichen Umgebungsbedingungen erlaubt. Eine Aufgabe kann gegebenenfalls auch darin gesehen werden, die mechanischen Eigenschaften von Papier und/oder papierähnlichen Materialien in Abhängigkeit von Temperatur und/oder relativer Luftfeuchte zu bestimmen. Eine noch weitere Aufgabe kann gegebenenfalls darin gesehen werden, die mechanischen Eigenschaften unterschiedlicher Papiermaterialien bei unterschiedlichen Umgebungsbedingungen, insbesondere Temperatur und/oder relativer Luftfeuchte, vergleichen zu können. Papier wird unter unterschiedlichen Bedingungen für unterschiedliche Zwecke verwendet. Jede Kombination erfordert gegebenenfalls eine gesonderte Untersuchung, um das bestmögliche Papier für die geforderte Zielanwendung zur Verfügung stellen zu können.

Gegebenenfalls wird zumindest eine der oben genannten Aufgaben mit einem erfindungsgemäßen Verfahren gelöst.

Die vorliegende Erfindung betrifft somit ein Verfahren zur Messung des Dehnungsverhaltens von Papier und/oder eines papierähnlichen Materials bei vorbestimmten, insbesondere unterschiedlichen, Umgebungsbedingungen. Unter die Definition eines "papierähnlichen Materials" fällt im Rahmen der vorliegenden Erfindung gegebenenfalls jeder flächige Werkstoff, der ein verdichtetes Faservliesmaterial enthält. Insbesondere umfasst ein "papierähnliches Material" im Sinne der vorliegenden Erfindung Pappe und Karton, sowie gegebenenfalls auch Pseudopapiere aus unterschiedlichen pflanzlichen Fasern. Ein "papierähnliches Material" im Sinne der vorliegenden Erfindung kann auch ein Papier sein, das mit einer Beschichtung aus natürlichen Polymeren, synthetischen Polymeren, Pigmenten, Metall oder einer Mischung aus mehreren der genannten Stoffe versehen ist.

Gegebenenfalls wird die Messung an einem Prüfkörper durchgeführt, der aus dem zu untersuchenden Material gebildet ist. Der Prüfkörper kann insbesondere ein flächiges Materialstück sein. In einer speziellen Ausführungsform ist der Prüfkörper ein flächiges Materialstück mit einer Länge von etwa 100 cm und einer Breite von etwa 20 cm.

Der Prüfkörper kann in einem Verfahrensschritt zwischen zwei gegenüberliegenden Halteeinrichtungen befestigt werden.

Zur Durchführung des Verfahrens kann eine vorbestimmte Umgebungsbedingung eingestellt werden. Die Umgebungsbedingung kann insbesondere eine vorbestimmte Temperatur und/oder eine vorbestimmte relative Luftfeuchte umfassen. Die Einstellung der Umgebungsbedingung kann beispielsweise mittels einer Klimaeinstellvorrichtung oder einer anderen Vorrichtung erfolgen, die dazu geeignet ist, eine entsprechende Umgebungsbedingung festzulegen. Beispielsweise kann das Verfahren in einer Klimakammer erfolgen, in der zumindest Temperatur und relative Luftfeuchte eingestellt werden können.

Die Umgebungsbedingung bezeichnet insbesondere jene Bedingung, der der Prüfkörper ausgesetzt ist oder wird. Beispielsweise bedeutet das Einstellen einer vorbestimmten Temperatur und/oder einer vorbestimmten Luftfeuchte, dass der Prüfkörper dieser vorbestimmten Temperatur und/oder dieser vorbestimmten Luftfeuchte ausgesetzt ist oder wird.

Nach dem Einstellen der Umgebungsbedingung kann eine Zugkraft auf den Prüfkörper ausgeübt werden. Insbesondere kann nach dem Einstellen der Umgebungsbedingung und vor dem Ausüben der Zugkraft eine Equilibrierungszeit vorgesehen sein, die derart bemessen ist, dass sich der Prüfkörper an die eingestellte Umgebungsbedingung anpassen kann. Dadurch kann sichergestellt werden, dass die Messung bei Gleichgewichtsbedingungen erfolgt. Die Equilibrierungszeit kann zumindest 4 Stunden, insbesondere zumindest 12 Stunden, gegebenenfalls zumindest 24 Stunden, betragen.

Die auf den Prüfkörper ausgeübte Zugkraft kann je nach Bedarf angepasst werden. Gegebenenfalls wird die Zugkraft während des Verfahrens konstant gehalten. Die Zugkraft kann gegebenenfalls auch verändert werden.

Gegebenenfalls liegt die auf den Prüfkörper ausgeübte Zugkraft zwischen 70 N/m und 1000 N/m.

Beim Ausüben der Zugkraft kann die Länge des Prüfkörpers im Vergleich zur Länge des Prüfkörpers vor Ausübung der Zugkraft bestimmt werden. Insbesondere wird also die durch die Ausübung der Zugkraft bedingte Änderung der Länge des Prüfkörpers bestimmt. Gegebenenfalls ist die Länge des Prüfkörpers vor Ausübung der Zugkraft die Länge des Prüfkörpers nach der Equilibrierungszeit, jedoch vor Ausübung der Zugkraft.

Die ausgeübte Zugkraft wirkt insbesondere im Wesentlichen nur in einer Raumrichtung. Eine derartige Zugkraft kann auch als monoaxiale Zugkraft bezeichnet werden. Die Zugkraft wirkt bevorzugt parallel zur Längserstreckungsrichtung des Prüfkörpers. Bevorzugt wirkt in Dickenrichtung des Prüfkörpers keine oder im Wesentlichen keine Zugkraft.

Die ausgeübte Zugkraft ist insbesondere kleiner als jene Zugkraft, die der Reißfestigkeit des Prüfkörpers entspricht. Dies bedeutet, dass der Prüfkörper bei der Messung nicht zerstört wird. Dadurch wird ermöglicht, eine Messung des selben Prüfkörpers bei unterschiedlichen Umgebungsbedingungen durchzuführen und so beispielsweise Rückschlüsse auf die temperatur- und/oder luftfeuchteabhängigen Dehnungseigenschaften des untersuchten Materials zu erhalten.

In einer speziellen Ausgestaltung wird das Verfahren mit mehreren Prüfkörpern durchgeführt bzw. wiederholt. Dabei stammen die mehreren Prüfkörper insbesondere aus demselben Material und weisen im Wesentlichen dieselbe Geometrie auf.

In diesem Zusammenhang bedeutet "aus demselben Material" insbesondere, dass sich die Materialeigenschaften der Prüfkörper bis auf übliche Schwankungen nicht voneinander unterscheiden. Beispielsweise würden mehrere Prüfkörper, die aus einem größeren Papierstück erhalten wurden, unter diese Definition fallen. Ferner würden auch mehrere Prüfkörper, die aus einem im Rahmen derselben Charge hergestellten Papiermaterial erhalten wurden, unter diese Definition fallen. "Im Wesentlichen dieselbe Geometrie" bedeutet in diesem Zusammenhang insbesondere, dass die mehreren Prüfkörper bis auf übliche Schwankungen dieselbe geometrische Form und dieselben Abmessungen, also Breite, Länge und Dicke, aufweisen.

Die Parameter Zugkraft, Temperatur und/oder relative Luftfeuchte können bei der wiederholten Durchführung der Verfahrensschritte an mehreren Prüfkörpern im Wesentlichen gleich bleiben. Dies ermöglicht die Bestimmung der Varianz der ermittelten Längenänderung.

Zumindest einer der Parameter aus Zugkraft, Temperatur und/oder relativer Luftfeuchte kann bei der wiederholten Durchführung der Verfahrensschritte an mehreren Prüfkörpern auch verändert werden. Dies ermöglicht die Bestimmung der Abhängigkeit der ermittelten Längenänderung in Bezug auf den zumindest einen veränderten Parameter.

Insbesondere wird genau ein Parameter verändert, während die anderen beiden Parameter gleich oder im Wesentlichen gleich bleiben.

Ein vollständiger Durchlauf der Verfahrensschritte kann auch als Prüfzyklus bezeichnet werden. Erfolgt eine zweimalige Wiederholung der Verfahrensschritte, werden also zwei Prüfzyklen durchgeführt.

In unterschiedlichen Prüfzyklen werden gegebenenfalls unterschiedliche Prüfkörper verwendet, die aus demselben Material stammen und im Wesentlichen dieselbe Geometrie aufweisen.

Gegebenenfalls wird die Änderung der Länge des Prüfkörpers in mehreren Prüfzyklen ermittelt, wobei die Zugkraft zwischen den unterschiedlichen Prüfzyklen verändert wird und die Temperatur sowie die relative Luftfeuchte gleich bleiben. Dies erlaubt die Bestimmung der Änderung der Länge des Prüfkörpers in Abhängigkeit von der Zugkraft.

Gegebenenfalls wird die Änderung der Länge des Prüfkörpers in mehreren Prüfzyklen ermittelt, wobei die Temperatur zwischen den unterschiedlichen Prüfzyklen verändert wird und die Zugkraft sowie die relative Luftfeuchte gleich bleiben. Dies erlaubt die Bestimmung der Änderung der Länge des Prüfkörpers in Abhängigkeit von der Temperatur.

Gegebenenfalls wird die Änderung der Länge des Prüfkörpers in mehreren Prüfzyklen ermittelt, wobei die relative Luftfeuchte zwischen den unterschiedlichen Prüfzyklen verändert wird und die Temperatur sowie die Zugkraft gleich bleiben. Dies erlaubt die Bestimmung der Änderung der Länge des Prüfkörpers in Abhängigkeit von der relativen Luftfeuchte.

Durch Korrelation der gemessenen Längenänderung in Abhängigkeit von den eingestellten Parametern können gegebenenfalls ein Zugkraft-Dehnungs-Koeffizient, ein Temperatur-Dehnungs-Koeffizient und/oder ein Luftfeuchte-Dehnungs-Koeffizient des Materials bestimmt werden. Die ermittelten Parameter können verwendet werden, um die Eigenschaften unterschiedlicher Materialien zu vergleichen.

Gegebenenfalls kann aus der in einem ersten Prüfzyklus gemessenen Änderung der Länge (ΔL₁) des Prüfkörpers und der im zweiten Prüfzyklus gemessenen Änderung der Länge (ΔL₂) des Prüfkörpers gemäß der Formel ΔL₁ / ΔL₂ ein Dehnungs-Koeffizient (K_{D}) berechnet werden. In diesem Fall werden insbesondere unterschiedliche Prüfkörper verwendet, die aus demselben Material stammen und im Wesentlichen dieselbe Geometrie aufweisen. Insbesondere ist die Wartezeit, nach der die Änderung der Länge der Prüfkörper in den beiden Prüfzyklen bestimmt wird, gleich. Bevorzugt ist zwischen erstem Prüfzyklus und zweitem Prüfzyklus genau ein Parameter aus Zugkraft; Temperatur und relativer Luftfeuchte unterschiedlich, während die anderen beiden Parameter gleich bleiben.

Die Bestimmung eines Dehnungs-Koeffizienten erlaubt den Vergleich unterschiedlicher Papiere oder papierähnlicher Materialien in Hinblick auf ihre Dehnungseigenschaften. Gegebenenfalls können bestimmte Verfahrensschritte auch an einem einzigen Prüfkörper mehrere Male durchgeführt bzw. wiederholt werden. So kann beispielsweise eine Messung der Änderung der Länge des Prüfkörpers bei vorbestimmter Temperatur, relativer Luftfeuchte und Zugkraft erfolgen und anschließend kann eine Messung der Änderung der Länge desselben Prüfkörpers bei geänderter Temperatur, relativer Luftfeuchte und/oder Zugkraft erfolgen. Auch hierdurch lassen sich Rückschlüsse über die von den umgebungsbedingten Materialeigenschaften gewinnen.

Bekannte Messvorrichtungen sind üblicherweise nicht für derartige Messverfahren geeignet, da sie nur schwer transportabel sind und zudem meist sehr empfindlich auf die Änderung von Umgebungsbedingungen reagieren.

Eine weitere Aufgabe kann daher gegebenenfalls darin gesehen werden, eine Vorrichtung zu schaffen, die in einem solchen Verfahren zur Messung des Dehnungsverhaltens von Papier eingesetzt werden kann.

Die vorliegende Erfindung betrifft somit auch eine Vorrichtung zur Messung des Dehnungsverhaltens von Papier und/oder eines papierähnlichen Materials.

Gegebenenfalls umfasst die Vorrichtung eine erste Halteeinrichtung und eine zweite Halteeinrichtung. In einer Ausführung können beide Halteeinrichtungen Klemmeinrichtungen sein, die dazu eingerichtet sind, den Prüfkörper festzuklemmen. Die Klemmeinrichtungen können beispielsweise zwei gegenüberliegende Haltebacken aufweisen.

Die Halteeinrichtungen können gegebenenfalls dazu eingerichtet sein, eine Zugkraft auf den Prüfkörper auszuüben. Dabei kann die Ausübung der Zugkraft durch eine Zugeinrichtung auf unterschiedliche Arten erfolgen, beispielsweise durch einen Antrieb und/oder durch ein Belastungsgewicht.

Die Verwendung eines Belastungsgewichts ist vorteilhaft, da dies einen besonders einfachen Aufbau der Vorrichtung erlaubt, wodurch auch vermieden werden kann, dass die Veränderung der Umgebungsbedingung zu einer Veränderung der auf den Prüfkörper ausgeübten Zugkraft führt. Zusätzlich ist die Vorrichtung dadurch einfacher transportierbar und gegebenenfalls auch ohne externe Energiezufuhr einsetzbar, was insbesondere beim Einsatz in einer Klimakammer vorteilhaft und somit auch zum energieeffizienten Betrieb der Vorrichtung zweckdienlich ist.

Um den Prüfkörper von der Zugkraft zu entlasten, kann im Fall der Verwendung eines Belastungsgewichts als Zugeinrichtung eine Entlastungseinrichtung vorgesehen sein, die dazu eingerichtet ist, den Prüfkörper zumindest teilweise von der Zugkraft zu entlasten.

Gegebenenfalls ist zur Messung der Änderung der Länge des Prüfkörpers eine Messeinrichtung vorgesehen. Die Messeinrichtung kann auf beliebige Weise ausgebildet sein, sofern diese zur Messung der Längenänderung geeignet ist. In einer Ausführung kann die Messeinrichtung als Messskala ausgebildet sein, die eine manuelle Ablesung des jeweiligen Messwerts erlaubt. Dies vereinfacht den Aufbau der Vorrichtung und erleichtert die Transportierbarkeit. Ferner wird dann auch keine externe Energiezufuhr für die Messeinrichtung benötigt, was, wie bereits erwähnt, einen sparsamen Energieeinsatz zum Betrieb der Anlage ermöglicht.

Die Erfindung betrifft gegebenenfalls auch die Verwendung einer erfindungsgemäßen Vorrichtung in einem erfindungsgemäßen Verfahren.

### Gegebenenfalls betrifft die Erfindung ein Verfahren zur Messung des Dehnungsverhaltens von Papier und/oder eines papierähnlichen Materials

Gegebenenfalls umfasst das Verfahren die folgenden Schritte
(a) Befestigen eines Prüfkörpers zwischen zwei gegenüberliegenden Halteeinrichtungen, wobei der Prüfkörper Papier und/oder ein papierähnliches Material umfasst oder daraus besteht,
(b) Einstellen einer vorbestimmten Temperatur und einer vorbestimmten relativen Luftfeuchte für die Messung,
(c) Ausüben einer Zugkraft auf den Prüfkörper, und
(d) Messen der Änderung der Länge des Prüfkörpers bei der auf den Prüfkörper ausgeübten Zugkraft.

Das Messen der Änderung der Länge des Prüfkörpers bei der auf den Prüfkörper ausgeübten Zugkraft direkt oder indirekt erfolgen, beispielsweise auch durch Berechnen einer Differenz aus zwei Längenmessungen.

Gegebenenfalls ist vorgesehen, dass die Schritte (a) bis (d) in einem ersten Prüfzyklus an einem ersten Prüfkörper durchgeführt werden, und dass die Schritte (a) bis (d) in einem zweiten Prüfzyklus an einem zweiten Prüfkörper durchgeführt werden, wobei im ersten Prüfzyklus im Vergleich zum zweiten Prüfzyklus zumindest einer der folgenden Parameter unterschiedlich ist: Temperatur, relative Luftfeuchte.

Gegebenenfalls ist vorgesehen, dass die Schritte (a) bis (d) in einem ersten Prüfzyklus an einem ersten Prüfkörper (3) durchgeführt werden, und dass die Schritte (a) bis (d) in einem zweiten Prüfzyklus an einem zweiten Prüfkörper (3) durchgeführt werden, wobei im ersten Prüfzyklus im Vergleich zum zweiten Prüfzyklus die Zugkraft unterschiedlich ist.

Gegebenenfalls ist vorgesehen, dass im ersten Prüfzyklus im Vergleich zum zweiten Prüfzyklus genau einer der folgenden Parameter unterschiedlich ist: Zugkraft, Temperatur, relative Luftfeuchte; und dass die verbleibenden Parameter im ersten Prüfzyklus und im zweiten Prüfzyklus im Wesentlichen gleich sind.

Gegebenenfalls ist vorgesehen, dass im ersten Prüfzyklus im Vergleich zum zweiten Prüfzyklus die Zugkraft unterschiedlich ist, und dass durch Korrelation von gemessener Änderung der Länge des ersten Prüfkörpers und gemessener Änderung der Länge des zweiten Prüfkörpers mittels der Formel K_{D(Fz)} = ΔL₁ / ΔL₂, wobei ΔL₁ die gemessene Änderung der Länge des ersten Prüfkörpers und ΔL₂ die gemessene Änderung der Länge des zweiten Prüfkörpers bezeichnen, ein Zugkraft-Dehnungs-Koeffizient des Materials ermittelt wird.

Gegebenenfalls ist vorgesehen, dass im ersten Prüfzyklus im Vergleich zum zweiten Prüfzyklus die Temperatur unterschiedlich ist, und dass durch Korrelation von gemessener Änderung der Länge des ersten Prüfkörpers und gemessener Änderung der Länge des zweiten Prüfkörpers mittels der Formel K_{D(T)} = ΔL₁ / ΔL₂, wobei ΔL₁ die gemessene Änderung der Länge des ersten Prüfkörpers und ΔL₂ die gemessene Änderung der Länge des zweiten Prüfkörpers bezeichnen, ein Temperatur-DehnungsKoeffizient des Materials ermittelt wird.

Gegebenenfalls ist vorgesehen, dass im ersten Prüfzyklus im Vergleich zum zweiten Prüfzyklus die relative Luftfeuchte unterschiedlich ist, und dass durch Korrelation von gemessener Änderung der Länge des ersten Prüfkörpers und gemessener Änderung der Länge des zweiten Prüfkörpers mittels der Formel K_{D(rH)} = ΔL₁ / ΔL₂, wobei ΔL₁ die gemessene Änderung der Länge des ersten Prüfkörpers und ΔL₂ die gemessene Änderung der Länge des zweiten Prüfkörpers bezeichnen, ein Luftfeuchte-Dehnungs-Koeffizient des Materials ermittelt wird.

Gegebenenfalls ist vorgesehen, dass die Schritte (a) bis (d) in einem ersten Prüfzyklus an einem ersten Prüfkörper durchgeführt werden, und dass die Schritte (a) bis (d) in einem zweiten Prüfzyklus an einem zweiten Prüfkörper durchgeführt werden, wobei der erste Prüfkörper und der zweite Prüfkörper aus demselben Material stammen und im Wesentlichen dieselbe Geometrie aufweisen.

Gegebenenfalls ist vorgesehen, dass die Schritte (c) und (d) bei einer ersten Umgebungsbedingung und bei einer zweiten Umgebungsbedingung durchgeführt werden, wobei sich die erste Umgebungsbedingung und die zweite Umgebungsbedingung durch die in Schritt (b) eingestellte Temperatur und/oder die eingestellte relative Luftfeuchte voneinander unterscheiden.

Gegebenenfalls ist vorgesehen, dass die Schritte (c) und (d) abwechselnd bei der ersten Umgebungsbedingung und bei der zweiten Umgebungsbedingung durchgeführt und bei jeder Umgebungsbedingung zumindest zweimal wiederholt werden.

Gegebenenfalls ist vorgesehen, dass die auf den Prüfkörper ausgeübte Zugkraft bei der ersten Umgebungsbedingung und bei der zweiten Umgebungsbedingung im Wesentlichen konstant ist.

Gegebenenfalls ist vorgesehen, dass während Schritt (c) die auf den Prüfkörper ausgeübte Zugkraft im Wesentlichen konstant ist, und dass während Schritt (c) die eingestellte Temperatur und/oder die eingestellte relative Luftfeuchte kontinuierlich oder inkrementell verändert wird.

Gegebenenfalls ist vorgesehen, dass die auf den Prüfkörper ausgeübte Zugkraft kleiner ist als jene Kraft, die der Reißfestigkeit des Prüfkörpers entspricht.

Gegebenenfalls ist vorgesehen, dass die auf den Prüfkörper ausgeübte Zugkraft kontinuierlich oder inkrementell verändert, insbesondere erhöht, wird.

Gegebenenfalls ist vorgesehen, dass dass die auf den Prüfkörper ausgeübte Zugkraft bis zum Erreichen einer vorbestimmten Änderung der Länge des Prüfkörpers kontinuierlich oder inkrementell erhöht wird.

Gegebenenfalls ist vorgesehen, dass das Einstellen der Temperatur und/oder der relativen Luftfeuchte durch eine Klimaeinstellvorrichtung erfolgt, wobei die Klimaeinstellvorrichtung gegebenenfalls eine Regeleinrichtung zur Regelung von Temperatur und/oder relativer Luftfeuchte umfasst.

Gegebenenfalls ist vorgesehen, dass das Messen der Änderung der Länge des Prüfkörpers in Schritt (d) nach Ablauf einer Wartezeit ab dem Ausüben der Zugkraft auf den Prüfkörper in Schritt (c) erfolgt, wobei die Wartezeit zwischen 1 Minute und 60 Minuten beträgt.

Gegebenenfalls betrifft die Erfindung eine Vorrichtung zur Messung des Dehnungsverhaltens von Papier und/oder eines papierähnlichen Materials.

Gegebenenfalls umfasst die Vorrichtung eine erste Halteeinrichtung und eine zweite Halteeinrichtung, wobei die Halteeinrichtungen dazu eingerichtet sind, einen Prüfkörper zu fixieren und eine Zugkraft auf den Prüfkörper auszuüben, wobei die Vorrichtung eine Messeinrichtung umfasst, die dazu eingerichtet ist, die Änderung der Länge des Prüfkörpers bei ausgeübter Zugkraft zu messen.

Gegebenenfalls ist vorgesehen, dass die erste Halteeinrichtung auf einem, insbesondere vertikal, verfahrbaren Schlitten angeordnet ist, und dass die zweite Halteeinrichtung gegebenenfalls stationär angeordnet ist.

Gegebenenfalls ist vorgesehen, dass die erste Halteeinrichtung eine Zugeinrichtung zum Ausüben der Zugkraft aufweist, wobei die Zugeinrichtung gegebenenfalls aus einer oder mehreren der folgenden Einrichtungen ausgewählt ist: Belastungsgewicht, pneumatischer Antrieb, hydraulischer Antrieb, elektrischer Antrieb, mechanischer Antrieb.

Gegebenenfalls ist vorgesehen, dass die Zugeinrichtung ein Belastungsgewicht ist oder ein Belastungsgewicht umfasst, das am Schlitten angeordnet ist, und dass eine Entlastungseinrichtung vorgesehen ist, um den Prüfkörper von der durch das Belastungsgewicht auf den Prüfkörper ausgeübten Zugkraft teilweise oder vollständig zu entlasten.

Gegebenenfalls ist vorgesehen, dass die Vorrichtung zur Umgebungsatmosphäre offen ausgebildet ist.

Gegebenenfalls ist vorgesehen, dass die Zugeinrichtung dazu eingerichtet ist, eine vertikal nach unten gerichtete, monoaxiale Zugkraft auf den Prüfkörper auszuüben.

Gegebenenfalls betrifft die Erfindung eine Anordnung einer erfindungsgemäßen Vorrichtung mit einem Prüfkörper, wobei der Prüfkörper Papier und/oder ein papierähnliches Material umfasst oder daraus besteht.

Gegebenenfalls betrifft die Erfindung die Verwendung einer erfindungsgemäßen Vorrichtung 20 zur Messung des Dehnungsverhaltens von Papier und/oder eines papierähnlichen Materials in einem erfindungsgemäßen Verfahren.

Weitere Merkmale der Erfindung ergeben sich aus den Patentansprüchen, den Figuren sowie aus der Beschreibung der Ausführungsbeispiele.

Nachfolgend wird die vorliegende Erfindung anhand von exemplarischen Ausführungsbeispielen im Detail erläutert. Die Ausführungsbeispiele illustrieren bevorzugte Merkmalskombinationen der vorliegenden Erfindung und sollen den Schutzumfang der Patentansprüche nicht einschränken.

Es zeigt:

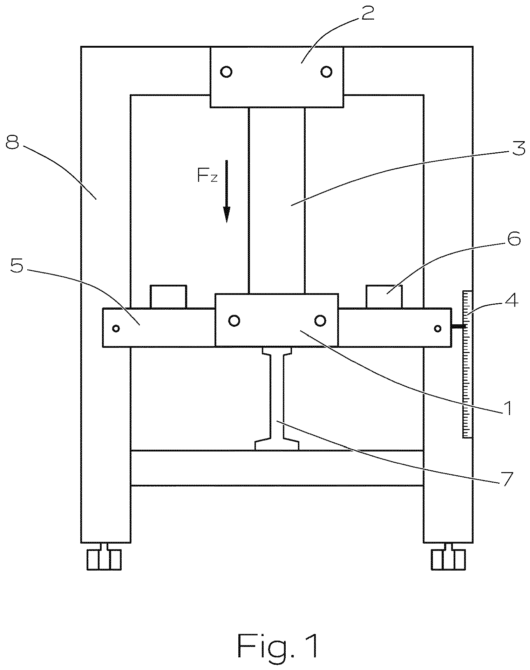

Fig. 1 eine schematische Darstellung eines Ausführungsbeispiels einer erfindungsgemäßen Vorrichtung.

Sofern nicht anders bezeichnet, zeigt die Figur die folgenden Merkmale: erste Halteeinrichtung 1, zweite Halteeinrichtung 2, Prüfkörper 3, Messeinrichtung 4, Schlitten 5, Zugeinrichtung 6, Entlastungseinrichtung 7, Rahmen 8.

Fig. 1 eine schematische Darstellung eines Ausführungsbeispiels einer erfindungsgemäßen Vorrichtung zur Messung des Dehnungsverhaltens von Papier und/oder eines papierähnlichen Materials.

Die in Fig. 1 gezeigte Vorrichtung umfasst eine erste Halteeinrichtung 1 sowie eine zweite Halteeinrichtung 2. Zwischen den Halteeinrichtungen 1, 2 ist ein Prüfkörper 3 angeordnet, der nachfolgend im Detail beschrieben ist.

Die Halteeinrichtungen 1, 2 weisen jeweils zwei gegenüberliegende Haltebacken (nicht dargestellt) auf, zwischen welchen der Prüfkörper 3 festgeklemmt wird. Während die zweite Halteeinrichtung 2 fest am Rahmen 8 der Vorrichtung angeordnet ist, ist die erste Halteeinrichtung an einem Schlitten 5 angeordnet, der relativ zum Rahmen 8 verschiebbar ist. Dadurch kann insbesondere eine Relativbewegung zwischen erster Halteeinrichtung 1 und zweiter Halteeinrichtung 2 erreicht werden. Der Schlitten 5 ist in vertikaler Richtung verschiebbar.

Am Schlitten 5 ist eine Zugeinrichtung 6 angeordnet, die in diesem Ausführungsbeispiel als Belastungsgewichte ausgebildet sind. Je nach aufzubringender Zugkraft F_{z} kann die Masse der Belastungsgewichte angepasst werden. Durch die vertikale Verschiebbarkeit des Schlittens 5 wirkt auch die Zugkraft F_{z} in vertikaler Richtung.

Die Vorrichtung umfasst ferner eine Entlastungseinrichtung 7, die den Schlitten 5 in einer festen Position halten kann. Dadurch wird die Möglichkeit geschaffen, den Prüfkörper 3 nur bei Bedarf mit der Zugkraft F_{z} zu belasten, nämlich wenn die Halteeinrichtung 7 entfernt oder gelöst wird.

Zur Messung der Länge des Prüfkörpers 3 bzw. zur Bestimmung der Änderung der Länge des Prüfkörpers 3 ist eine Messeinrichtung 4 vorgesehen. Die Messeinrichtung 4 umfasst in diesem Ausführungsbeispiel eine fest am Rahmen 8 angeordnete Messskala sowie einen am Schlitten 5 angeordneten Messzeiger, sodass der Messwert auf der Skala abgelesen werden kann.

Der Rahmen 8 ist zur Umgebung offen ausgebildet, sodass eine rasche Anpassung an die Umgebungsbedingungen ermöglicht wird. Dies erlaubt in vorteilhafter Weise den Einsatz der Vorrichtung beispielsweise in einer Klimakammer.

In weiteren Versuchen wurde das Dehnungsverhalten von Kraftpapier mittels der in Fig. 1 gezeigten Vorrichtung untersucht. Dafür wurde ein Ausführungsbeispiel eines erfindungsgemäßen Verfahrens angewendet.

Als Prüfkörper 3 wurden rechteeckige Stücke eines Kraftpapiers mit etwa 120 cm Länge und etwa 20 cm Breite eingesetzt. Alle Prüfkörper 3 stammten aus derselben Herstellungscharge. Die Grammatur des Kraftpapiers betrug etwa 80 g/m².

Jeweils ein Prüfkörper wurde zur Durchführung eines Prüfzyklus in der Vorrichtung befestigt, wobei das Festklemmen in erster und zweiter Halteeinrichtung 1, 2 entlang der gesamten Breite des jeweiligen Prüfkörpers 3 mit einer Klemmkraft von etwa 0,3 kN erfolgte. Ein Bereich des Prüfkörpers 3 von etwa 5 cm Höhe wurde in jeder Halteeinrichtung 1, 2 eingeklemmt, sodass die freie Länge des Prüfkörpers 3 zwischen den beiden Halteeinrichtungen 1, 2 etwa 100 cm betrug; die restlichen 10 cm des Prüfkörpers 3 ragten über die Halteeinrichtung 2 hinaus.

Das Einstellen der Umweltbedingung, also der Temperatur und der relativen Luftfeuchte erfolgte in einer Klimakammer, in der die Vorrichtung angeordnet wurde.

Nach dem Einstellen von Temperatur und relativer Luftfeuchte und vor dem Ausüben der Zugkraft F_{z} auf den Prüfkörper 3 wurde eine Equilibrierungszeit von zumindest 24 Stunden vorgesehen, um den Prüfkörper 3 in Gleichgewichtsbedingung zu bringen. Die Equilibrierung erfolgte derart, dass die Prüfkörper 3 in der Klimakammer ausgelegt wurden und während der Equilibrierungszeit in spannungsfreiem Zustand dort belassen wurden. Die Equilibrierung erfolgte somit vor dem Einspannen der Prüfkörper 3 in die Prüfvorrichtung. Die eingestellte Temperatur und relative Luftfeuchte entsprechen der ersten Umgebungsbedingung.

Nach dem Ablauf der Equilibrierungszeit wurde die initiale Länge des Prüfkörpers 3, auch als L₀ bezeichnet, bestimmt. L₀ entspricht also jener Länge des Prüfkörpers 3 ohne Zugbelastung, aber bei Gleichgewichtsbedingungen vorbestimmter Temperatur und vorbestimmter relativer Luftfeuchte. L₀ wurde durch Ablesen des entsprechenden Werts auf der Messeinrichtung 4 ermittelt.

Das Ausüben der Zugkraft F_{z} wurde durch Entfernen der Entlastungseinrichtung 7 gestartet. Während der Durchführung eines Messzyklus erfolgte keine Änderung von Zugkraft F_{z}, Temperatur und relativer Luftfeuchte.

Nach unterschiedlichen Zeitpunkten, nämlich nach einer Wartezeit von etwa 1, 5, 10, 30 und 60 Minuten, nach Aufbringen der Zugkraft F_{z} wurde die Länge des Prüfkörpers 3, auch als L bezeichnet, bestimmt. L entspricht also jener Länge des Prüfkörpers 3 mit Zugbelastung bei vorbestimmter Temperatur und vorbestimmter relativer Luftfeuchte. L wurde durch Ablesen des entsprechenden Werts auf der Messeinrichtung 4 ermittelt.

Die Änderung der Länge des Prüfkörpers 3 bei der Zugkraft F_{z}, auch als ΔL bezeichnet, wurde durch Berechnung der Differenz zwischen L und L₀ bzw. nach der folgenden Gleichung berechnet: ΔL = L - L₀.

Nach Durchführung der Messung bei der ersten Umgebungsbedingung, also nach Ablauf der längsten Wartezeit von etwa 60 Minuten, wurde die Prüfvorrichtung mit dem Prüfkörper 3 in eine zweite Klimakammer gebracht. In der zweiten Klimakammer herrschte eine zweite Umgebungsbedingung, die sich in Temperatur und relativer Luftfeuchte von der ersten Umgebungsbedingung unterschied.

Bei der zweiten Umgebungsbedingung wurden wiederum Messungen der Länge L des Prüfkörpers 3 nach einer Wartezeit von etwa 1, 5, 10, 30 und 60 Minuten durchgeführt.

Die Wartezeit wurde ab der Platzierung der Prüfvorrichtung in der zweiten Klimakammer gemessen. Nach Ablauf der längsten Wartezeit von 60 Minuten wurde der Prüfkörper 3 entlastet und aus der Halteeinrichtung 1, 2 entfernt.

Weitere Prüfzyklen wurden mit neuen Prüfkörpern 3 durchgeführt.

Aus den durchgeführten Prüfzyklen wurden die nachfolgend in den Tabellen 1-4 dargestellten Längenänderungen ΔL in Abhängigkeit von Zugkraft F_{z}, Temperatur T und relativer Luftfeuchte rH erhalten.

Die Tabellen 1-4 zeigen die in unterschiedlichen Prüfzyklen gemessene Längenänderung ΔL der Prüfkörper 3 bei der ersten und der zweiten Umgebungsbedingung, mit ΔL = L - L₀ und L₀ = 100 cm.

Die in den Tabellen 1-4 angegebene Schrumpfung bezeichnet die Änderung der Länge des Prüfkörpers 3 bei der zweiten Umgebungsbedingung in Bezug auf die Länge des Prüfkörpers 3 nach Ablauf der längsten Wartezeit von 60 Minuten bei der ersten Umgebungsbedingung.

**Tabelle 1: Längenänderung eines Prüfkörpers in einem ersten Prüfzyklus bei erster und zweiter Umgebungsbedingung**

| | | | |
|---|---|---|---|
| **Umgebungsbedingung Nr.** | 1 | 2 | |
| **Zugkraft F_{z} (N)** | 72,6 | | |
| **Temperatur T (°C)** | 23 | 30 | |
| **relative Luftfeuchte rH (%)** | 50 | 22 | |

| **Wartezeit (min)** | **Längenänderung ΔL (mm)** | | **Schrumpfung (mm)** |
|---|---|---|---|
| 1 | 3,0 | 2,5 | - 0,5 |
| 5 | 3,0 | 2,0 | - 1,0 |
| 10 | 3,0 | 2,0 | - 1,0 |
| 30 | 3,0 | 2,0 | - 1,0 |
| 60 | 3,0 | 1,5 | - 1,5 |

**Tabelle 2: Längenänderung eines Prüfkörpers in einem zweiten Prüfzyklus bei erster und zweiter Umgebungsbedingung**

| | | | |
|---|---|---|---|
| **Umgebungsbedingung Nr.** | 1 | 2 | |
| **Zugkraft F_{z} (N)** | 72,6 | | |
| **Temperatur T (°C)** | 30 | 23 | |
| **relative Luftfeuchte rH (%)** | 50 | 35 | |

| **Wartezeit (min)** | **Längenänderung ΔL (mm)** | | **Schrumpfung (mm)** |
|---|---|---|---|
| 1 | 3,0 | 3,0 | - 1,0 |
| 5 | 3,0 | 3,0 | - 1,0 |
| 10 | 3,0 | 3,0 | - 1,0 |
| 30 | 3,5 | 3,0 | - 1,0 |
| 60 | 4,0 | 3,0 | - 1,0 |

**Tabelle 3: Längenänderung eines Prüfkörpers in einem dritten Prüfzyklus bei erster und zweiter Umgebungsbedingung**

| | | | |
|---|---|---|---|
| **Umgebungsbedingung Nr.** | 1 | 2 | |
| **Zugkraft F_{z} (N)** | 100 | | |
| **Temperatur T (°C)** | 23 | 30 | |
| **relative Luftfeuchte rH (%)** | 50 | 24 | |

| **Wartezeit (min)** | **Längenänderung ΔL (mm)** | | **Schrumpfung (mm)** |
|---|---|---|---|
| 1 | 3,0 | 2,5 | - 0,5 |
| 5 | 3,0 | 2,0 | - 1,0 |
| 10 | 3,0 | 2,0 | - 1,0 |
| 30 | 3,0 | 2,0 | - 1,0 |
| 60 | 3,0 | 2,0 | - 1,0 |

**Tabelle 4: Längenänderung eines Prüfkörpers in einem vierten Prüfzyklus bei erster und zweiter Umgebungsbedingung**

| | | | |
|---|---|---|---|
| **Umgebungsbedingung Nr.** | 1 | 2 | |
| **Zugkraft F_{z} (N)** | 100 | | |
| **Temperatur T (°C)** | 30 | 27 | |
| **relative Luftfeuchte rH (%)** | 50 | 28 | |

| **Wartezeit (min)** | **Längenänderung ΔL (mm)** | | **Schrumpfung (mm)** |
|---|---|---|---|
| 1 | 3,0 | 5,0 | - 1,5 |
| 5 | 4,0 | 4,5 | -2,0 |
| 10 | 5,0 | 4,5 | - 2,0 |
| 30 | 6,0 | 4,0 | - 2,5 |
| 60 | 6,5 | 4,0 | - 2,5 |

Aus den erhaltenen Messwerten konnten Rückschlüsse auf die temperatur- und luftfeuchteabhängigen Materialeigenschaften des Kraftpapiers gewonnen werden.

Beispielsweise ist aus dem Vergleich zwischen den Tabellen 1 und 2 zu erkennen, dass die Prüfkörper bei gleicher Zugkraft, gleicher relativer Luftfeuchte und höherer Temperatur eine größere Längenänderung erfahren. Dies zeigt sich insbesondere bei längeren Wartezeiten, wie im Vergleich zwischen Prüfzyklus 3 (Tabelle 3) und 4 (Tabelle 4) bei einer Wartezeit von jeweils 60 Minuten ersichtlich ist. Ein ähnlicher Trend zeigt sich bei Vergleich von Prüfzyklus 1 (Tabelle 1) und 2 (Tabelle 2).

Bei Messpunkten gleicher Wartezeit lässt sich ein Dehnungs-Koeffizient K_{D} gemäß der Formel K_{D} = ΔL₁ / ΔL₂ berechnen. Dabei entsprechen ΔL₁ und ΔL₂ der gemessenen Längenänderung des Prüfkörpers bei unterschiedlichen Bedingungen, wobei sich bei den Messpunkten von ΔL₁ und ΔL₂ nur einer der Parameter aus Zugkraft, Temperatur und relativer Luftfeuchte unterscheidet und die jeweils anderen Parameter konstant sind. Je nach sich unterscheidendem Parameter wird der Dehnungskoeffizient K_{D} als Zugkraft-Dehnungs-Koeffizient K_{D(Fz)}, als Temperatur-Dehnungs-Koeffizient K_{D(T)} oder als Luftfeuchte-Dehnungs-Koeffizient K_{D(rH)} bezeichnet.

Beispielsweise beträgt im vorliegenden Beispiel der Temperatur-Dehnungs-Koeffizient K_{D(T)} definiert durch den Quotienten aus Längenänderung bei 23°C und Längenänderung bei 30°C bei einer Wartezeit von 60 Minuten, einer Zugkraft von 100 N und einer relativen Luftfeuchte von 50% etwa 0,46.

Der Temperatur-Dehnungs-Koeffizient K_{D(T)} definiert durch den Quotienten aus Längenänderung bei 23°C und Längenänderung bei 30°C bei einer Wartezeit von 60 Minuten, einer Zugkraft von 72,6 N und einer relativen Luftfeuchte von 50% beträgt etwa 0,75.

Mittels der Dehnungs-Koeffizienten können die entsprechenden Eigenschaften unterschiedlicher Prüfkörper bzw. Materialien miteinander verglichen werden. Beispielsweise erlaubt der Temperatur-Dehnungs-Koeffizient K_{D(T)} Rückschlüsse über die Abhängigkeit der Dehnung einer bestimmten Papiersorte in Bezug auf die Temperatur und/oder in Bezug auf Parameter, die mit der Temperatur in Verbindung stehen. Ein Parameter, der mit der Temperatur in Verbindung steht ist beispielsweise die absolute Luftfeuchte, die sich bei unterschiedlichen Temperaturen und konstanter relativer Luftfeuchte ändert.

Aus dem Vergleich zwischen zwei Umweltbedingungen, die innerhalb eines Messzyklus untersucht wurden, können ebenfalls Dehnungs-Koeffizienten abgeleitet werden.

## Patentansprüche

1. **Verfahren** zur Messung des Dehnungsverhaltens von Papier und/oder eines papierähnlichen Materials, umfassend die folgenden Schritte:
a. Befestigen eines Prüfkörpers (3) zwischen zwei gegenüberliegenden Halteeinrichtungen (1, 2), wobei der Prüfkörper (3) Papier und/oder ein papierähnliches Material umfasst oder daraus besteht,
b. Einstellen einer vorbestimmten Temperatur und einer vorbestimmten relativen Luftfeuchte für die Messung,
c. Ausüben einer Zugkraft (F_{z}) auf den Prüfkörper (3), und
d. Messen der Änderung der Länge des Prüfkörpers (3) bei der auf den Prüfkörper (3) ausgeübten Zugkraft (F_{z}).

2. Verfahren nach Anspruch 1, **dadurch gekennzeichnet,**
- **dass** die Schritte (a) bis (d) in einem ersten Prüfzyklus an einem ersten Prüfkörper (3) durchgeführt werden, und dass die Schritte (a) bis (d) in einem zweiten Prüfzyklus an einem zweiten Prüfkörper (3) durchgeführt werden, wobei im ersten Prüfzyklus im Vergleich zum zweiten Prüfzyklus zumindest einer der folgenden Parameter unterschiedlich ist: Temperatur, relative Luftfeuchte, und/oder
- **dass** die Schritte (a) bis (d) in einem ersten Prüfzyklus an einem ersten Prüfkörper (3) durchgeführt werden, und dass die Schritte (a) bis (d) in einem zweiten Prüfzyklus an einem zweiten Prüfkörper (3) durchgeführt werden, wobei im ersten Prüfzyklus im Vergleich zum zweiten Prüfzyklus die Zugkraft (F_{z}) unterschiedlich ist.

3. Verfahren nach Anspruch 1 oder 2, **dadurch gekennzeichnet, dass** im ersten Prüfzyklus im Vergleich zum zweiten Prüfzyklus genau einer der folgenden Parameter unterschiedlich ist: Zugkraft (F_{z}), Temperatur, relative Luftfeuchte; und dass die verbleibenden Parameter im ersten Prüfzyklus und im zweiten Prüfzyklus im Wesentlichen gleich sind.

4. Verfahren nach Anspruch 3, **dadurch gekennzeichnet,**
- **dass** im ersten Prüfzyklus im Vergleich zum zweiten Prüfzyklus die Zugkraft (F_{z}) unterschiedlich ist, und dass durch Korrelation von gemessener Änderung der Länge (ΔL₁) des ersten Prüfkörpers (3) und gemessener Änderung der Länge (ΔL₂) des zweiten Prüfkörpers (3) mittels der Formel K_{D(Fz)} = ΔL₁ / ΔL₂ ein Zugkraft-Dehnungs-Koeffizient des Materials ermittelt wird,
- oder dass im ersten Prüfzyklus im Vergleich zum zweiten Prüfzyklus die Temperatur unterschiedlich ist, und dass durch Korrelation von gemessener Änderung der Länge (ΔL₁) des ersten Prüfkörpers (3) und gemessener Änderung der Länge (ΔL₂) des zweiten Prüfkörpers (3) mittels der Formel K_{D(T)} = ΔL₁ / ΔL₂ ein Temperatur-Dehnungs-Koeffizient des Materials ermittelt wird,
- oder dass im ersten Prüfzyklus im Vergleich zum zweiten Prüfzyklus die relative Luftfeuchte unterschiedlich ist, und dass durch Korrelation von gemessener Änderung der Länge (ΔL₁) des ersten Prüfkörpers (3) und gemessener Änderung der Länge (ΔL₂) des zweiten Prüfkörpers (3) mittels der Formel K_{D(rH)} = ΔL₁ / ΔL₂ ein Luftfeuchte-Dehnungs-Koeffizient des Materials ermittelt wird.

5. Verfahren nach einem der Ansprüche 1 bis 4, **dadurch gekennzeichnet,**
- **dass** die Schritte (a) bis (d) in einem ersten Prüfzyklus an einem ersten Prüfkörper (3) durchgeführt werden, und dass die Schritte (a) bis (d) in einem zweiten Prüfzyklus an einem zweiten Prüfkörper (3) durchgeführt werden, wobei der erste Prüfkörper (3) und der zweite Prüfkörper (3) aus demselben Material stammen und im Wesentlichen dieselbe Geometrie aufweisen, und/oder
- **dass** die Schritte (c) und (d) bei einer ersten Umgebungsbedingung und bei einer zweiten Umgebungsbedingung durchgeführt werden, wobei sich die erste Umgebungsbedingung und die zweite Umgebungsbedingung durch die in Schritt (b) eingestellte Temperatur und/oder die eingestellte relative Luftfeuchte voneinander unterscheiden.

6. Verfahren nach Anspruch 5, **dadurch gekennzeichnet, dass** die Schritte (c) und (d) abwechselnd bei der ersten Umgebungsbedingung und bei der zweiten Umgebungsbedingung durchgeführt und bei jeder Umgebungsbedingung zumindest zweimal wiederholt werden, und/oder dass die auf den Prüfkörper (3) ausgeübte Zugkraft (F_{z}) bei der ersten Umgebungsbedingung und bei der zweiten Umgebungsbedingung im Wesentlichen konstant ist.

7. Verfahren nach einem der Ansprüche 1 bis 6, **dadurch gekennzeichnet, dass** während Schritt (c) die auf den Prüfkörper (3) ausgeübte Zugkraft (F_{z}) im Wesentlichen konstant ist, und dass während Schritt (c) die eingestellte Temperatur und/oder die eingestellte relative Luftfeuchte kontinuierlich oder inkrementell verändert wird.

8. Verfahren nach einem der Ansprüche 1 bis 7, **dadurch gekennzeichnet, dass** die auf den Prüfkörper (3) ausgeübte Zugkraft (F_{z}) kleiner ist als jene Kraft, die der Reißfestigkeit des Prüfkörpers (3) entspricht, und/oder dass die auf den Prüfkörper (3) ausgeübte Zugkraft (F_{z}) kontinuierlich oder inkrementell verändert, insbesondere erhöht, wird.

9. Verfahren nach einem der Ansprüche 1 bis 8, **dadurch gekennzeichnet, dass** die auf den Prüfkörper (3) ausgeübte Zugkraft (F_{z}) bis zum Erreichen einer vorbestimmten Änderung der Länge des Prüfkörpers (3) kontinuierlich oder inkrementell erhöht wird, und/oder dass das Einstellen der Temperatur und/oder der relativen Luftfeuchte durch eine Klimaeinstellvorrichtung erfolgt, wobei die Klimaeinstellvorrichtung gegebenenfalls eine Regeleinrichtung zur Regelung von Temperatur und/oder relativer Luftfeuchte umfasst.

10. Verfahren nach einem der Ansprüche 1 bis 9, **dadurch gekennzeichnet, dass** das Messen der Änderung der Länge des Prüfkörpers (3) in Schritt (d) nach Ablauf einer Wartezeit ab dem Ausüben der Zugkraft (F_{z}) auf den Prüfkörper (3) in Schritt (c) erfolgt, wobei die Wartezeit zwischen 1 Minute und 60 Minuten beträgt.

11. **Vorrichtung** zur Messung des Dehnungsverhaltens von Papier und/oder eines papierähnlichen Materials, umfassend eine erste Halteeinrichtung (1) und eine zweite Halteeinrichtung (2), wobei die Halteeinrichtungen (1, 2) dazu eingerichtet sind, einen Prüfkörper (3) zu fixieren und eine Zugkraft (F_{z}) auf den Prüfkörper (3) auszuüben, wobei die Vorrichtung eine Messeinrichtung (4) umfasst, die dazu eingerichtet ist, die Änderung der Länge des Prüfkörpers (3) bei ausgeübter Zugkraft (F_{z}) zu messen.

12. Vorrichtung nach Anspruch 11, **dadurch gekennzeichnet,**
- **dass** die erste Halteeinrichtung (1) auf einem, insbesondere vertikal, verfahrbaren Schlitten (5) angeordnet ist, und dass die zweite Halteeinrichtung (2) gegebenenfalls stationär angeordnet ist, und/oder
- **dass** die erste Halteeinrichtung (1) eine Zugeinrichtung (6) zum Ausüben der Zugkraft (F_{z}) aufweist, wobei die Zugeinrichtung (6) gegebenenfalls aus einer oder mehreren der folgenden Einrichtungen ausgewählt ist:
Belastungsgewicht, pneumatischer Antrieb, hydraulischer Antrieb, elektrischer Antrieb, mechanischer Antrieb.

13. Vorrichtung nach Anspruch 11 oder 12, **dadurch gekennzeichnet, dass** die Zugeinrichtung (6) ein Belastungsgewicht ist oder ein Belastungsgewicht umfasst, das am Schlitten (5) angeordnet ist, und dass eine Entlastungseinrichtung (7) vorgesehen ist, die dazu eingerichtet ist, den Prüfkörper (3) von der durch das Belastungsgewicht auf den Prüfkörper (3) ausgeübten Zugkraft (F_{z}) teilweise oder vollständig zu entlasten.

14. Vorrichtung nach einem der Ansprüche 11 bis 13, **dadurch gekennzeichnet, dass** die Vorrichtung zur Umgebungsatmosphäre offen ausgebildet ist, und/oder dass die Zugeinrichtung (6) dazu eingerichtet ist, eine vertikal nach unten gerichtete, monoaxiale Zugkraft (F_{z}) auf den Prüfkörper (3) auszuüben.

15. **Anordnung** einer Vorrichtung nach einem der Ansprüche 11 bis 14 mit einem Prüfkörper (3), wobei der Prüfkörper (3) Papier und/oder ein papierähnliches Material umfasst oder daraus besteht.

16. **Verwendung** einer Vorrichtung nach einem der Ansprüche 11 bis 14 zur Messung des Dehnungsverhaltens von Papier und/oder eines papierähnlichen Materials in einem Verfahren nach einem der Ansprüche 1 bis 10.
